(19)

Europäisches
Patentamt

European
Patent Office

Office européen
des brevets

(11) **EP 2 689 725 A1**

(12) **EUROPEAN PATENT APPLICATION**
published in accordance with Art. 153(4) EPC

(43) Date of publication:
    **29.01.2014 Bulletin 2014/05**

(21) Application number: **12760910.5**

(22) Date of filing: **23.03.2012**

(51) Int Cl.:
    **A61B 6/00** (2006.01)          **G06T 5/00** (2006.01)
    **G06T 5/20** (2006.01)

(86) International application number:
    **PCT/JP2012/002010**

(87) International publication number:
    **WO 2012/127872 (27.09.2012 Gazette 2012/39)**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**

(30) Priority: **24.03.2011   JP 2011065511**

(71) Applicant: **FUJIFILM Corporation**
    **Tokyo 106-8620 (JP)**

(72) Inventor: **IMAI, Yoshiro**
    **Ashigarakami-gun**
    **Kanagawa 258-8538 (JP)**

(74) Representative: **Klunker . Schmitt-Nilson . Hirsch**
    **Patentanwälte**
    **Destouchesstrasse 68**
    **80796 München (DE)**

(54) **IMAGE PROCESSING DEVICE, IMAGE PROCESSING METHOD, AND IMAGE PROCESSING PROGRAM**

(57)    [Aim] To generate a high quality image in which a periodic pattern, such as a folding distortion component, which is caused by a spatial frequency component of a grid image, is suppressed while a subject component is maintained in an image signal generation method and apparatus that generates an image signal by reading a storable phosphor sheet on which a radiographic image including the grid image is recorded.

[Solution Means] A first processed signal (Sh) is ex-tracted by extracting an input spatial frequency component corresponding to a periodic pattern by performing filtering processing on an image signal. A subject com-ponent (Sc) of an image included in the extracted first processed signal is extracted from the first processed signal. A second processed signal (Sp) is extracted by subtracting the first processed signal (Sh) from the image signal (Sd) and also by adding the extracted subject com-ponent (Sc) to the image signal (Sd).

FIG.5

EP 2 689 725 A1

**Description**

BACKGROUND OF THE INVENTION

Field of the Invention

The present invention relates to a periodic pattern suppression method and apparatus that suppresses a spatial frequency component corresponding to a periodic pattern in an image signal.

Description of the Related Art

Conventionally, storable phosphors (photostimulable phosphors) have been used. When a storable phosphor is irradiated with radiation (X-rays, α-rays, β-rays, γ-rays, an electron beam, ultraviolet rays or the like), a part of radiation energy is stored in the storable phosphor. After then, when excitation light, such as visible light and a laser beam, is output to the storable phosphor, the storable phosphor emits photoluminescence corresponding to the radiation energy stored therein. For example, a radiographic image readout apparatus using the storable phosphor is widely used in CR (Computed Radiography). A storable phosphor sheet, in which the storable phosphor is deposited on a substrate, is irradiated with radiation that has passed through a subject, such as a human body, and radiographic information is temporarily stored and recorded on the storable phosphor sheet. Excitation light, such as a laser beam, is output to the storable phosphor sheet to induce photoluminescence. Further, photoelectric conversion is performed on the photoluminescence to obtain an image signal.

Here, when a radiographic image of a subject is imaged and recorded on the storable phosphor sheet or the like, imaging is performed by placing a grid between the subject and the sheet in some cases so that radiation scattered by the subject does not irradiate the sheet. For example, lead or the like, which does not pass radiation therethrough, and aluminum, wood or the like, which tends to pass radiation therethrough, are alternately arranged at a narrow pitch of 4 line/mm in the grid. When radiography is performed by using the grid, radiation scattered by the subject does not tend to irradiate the sheet. Therefore, it is possible to improve the contrast of the radiographic image of the subject. However, when the size of this image including the grid image is enlarged or reduced, aliasing due to folding occurs depending on the magnification or reduction ratio. Further, when aliasing overlaps with the spatial frequency of the grid image or the like, a narrow stripe pattern (moire) is generated, and observation of a regenerated image becomes difficult.

Patent Document 1 discloses a method for removing a grid image, as a method for removing such a periodic pattern from an image. In the method for removing the grid image, one-dimensional high-pass filter processing is performed on the image in a direction in which the stripe pattern of the grid image is arranged. Further, one-dimensional low-pass filter processing is performed on the image in a direction parallel to the stripe pattern of the grid image. Accordingly, a spatial frequency component corresponding to the grid image is extracted from an original image, and the extracted spatial frequency component is subtracted from the original image.

Patent Document 2 proposes a method for obtaining an image in which an artifact caused by a grid has been effectively removed in radiographic image processing, in which radiographic image data of a subject obtained by performing radiography using the grid for removing radiation scattered by the subject are processed. First, data (band component) representing a first image component, in other words, a grid component are extracted from radiographic image data of the subject including the grid component. Then, second image component data, which are caused by the grid included in the radiographic image data, are generated based on the first image component data. The generated second image component data, in other words, data of a component caused by the grid are removed from original radiographic image data, in other words, the whole image including the grid component.

Patent Document 3 discloses a method for removing a grid. A band-pass grid image, which has been extracted from an original image by a band-pass filter, and a high-pass grid image, which has been extracted from the original image by a high-pass filter, are obtained to subtract a remaining grid image, which has not been removed as a grid image, from an original image. A difference grid image (a grid frequency component that has not been fully extracted by band-pass) is obtained by performing low-pass processing on a difference image, which is a difference between the two grid images, in a direction parallel to a grid pattern. An image obtained by adding the difference grid image and the band-pass grid image together is subtracted from the original image.

Related Technical Documents

Patent Documents

Patent Document 1:

Japanese Unexamined Patent Publication No. 2003-150954

Patent Document 2:

Japanese Patent No. 3445258

Patent Document 3:

Japanese Unexamined Patent Publication No. 2010-240259

SUMMARY OF THE INVENTION

Here, there is a demand for removing a periodic pattern, such as moire, without removing a component representing a subject as possible to obtain a high quality image appropriate for diagnosis based on the image. However, in the methods disclosed in Patent Documents 1 through 3, when a detected spatial frequency component in a predetermined band corresponding to a periodic pattern is removed from an image, there has been a problem that a subject component representing a subject, and which is included in the predetermined band, is removed together. Especially, when a moire component caused by a grid is present in a low spatial frequency band including many subject components representing the subject, the quality of an image deteriorates significantly in the methods disclosed in Patent Documents 1 through 3.

In view of the foregoing circumstances, it is an object of the present invention to provide an image processing apparatus, an  image processing method and an image processing program that can extract a subject component from a frequency component corresponding to a periodic pattern, and restore the extracted subject component to a current image.

An image processing apparatus of the present invention is an image processing apparatus that suppresses a spatial frequency component corresponding to a periodic pattern included in an image, the apparatus comprising:

a first processed signal extraction means that extracts a first processed signal by extracting an input spatial frequency component corresponding to the periodic pattern by performing filtering processing on the image signal;
a subject component extraction means that extracts, from the extracted first processed signal, a subject component of the image, and the subject component being included in the first processed signal; and
a second processed signal extraction means that extracts a second processed signal by subtracting the first processed signal from the image signal and also by adding the extracted subject component to the image signal.

An image processing method of the present invention is an image processing method that suppresses a spatial frequency component corresponding to a periodic pattern included in an image, the method comprising the steps of:

extracting a first processed signal by extracting an input spatial frequency component corresponding to the periodic pattern by performing filtering processing on the image signal; and
extracting a second processed signal by subtracting the first processed signal from the image signal and also by adding the extracted subject component to the image signal.

An image processing program of the present invention is an image processing program that suppresses a spatial frequency component corresponding to a periodic pattern included in an image, the program causing a computer to function as:

a first processed signal extraction means that extracts a first processed signal by extracting an input spatial frequency component corresponding to the periodic pattern by performing filtering processing on the image signal;
a subject component extraction means that extracts, from the extracted first processed signal, a subject component of the image, and the subject component being included in the first processed signal; and
a second processed signal extraction means that extracts a second processed signal by subtracting the first processed signal from the image signal and also by adding the extracted subject component to the image signal.

The term "periodic pattern" means a noise having a periodic pattern included in an original image. For example, the periodic pattern means a grid image, moire or the like included in an original image when a radiographic image is imaged on a storable phosphor sheet by using the grid.

Further, the "input" spatial frequency component corresponding to the periodic pattern includes a spatial frequency component corresponding to the periodic pattern, and which has been input by any method. For example, a known spatial frequency component corresponding to a periodic pattern may be input by a user's manual input. Alternatively, the image processing apparatus may further include a periodic pattern detection means that detects, in the image signal, a spatial frequency component corresponding to the periodic pattern, and that inputs the detected spatial frequency component to the first processed signal extraction means. Further, the spatial frequency component corresponding to the periodic pattern, and which has been detected by the periodic pattern detection means, may be input.

In the image processing apparatus of the present invention, it is desirable that the subject component extraction means extracts, from the first processed signal, a high contrast component exceeding a predetermined threshold, as the subject component.

Here, the term "contrast" means a difference in density values between a light part and a dark part in an image, and which are represented by density values of the image. The term "high contrast" means that the absolute value of a difference in density values from a neighboring pixel is greater than a predetermined threshold.

The threshold may differ depending on an imaged region or an imaging condition of the image. Here, an imaged region of an image and an imaging condition of the image may be obtained by using an imaging menu, such as information about the imaging condition and the imaged region, which is specified at an imaging apparatus of an original image. Alternatively, imaged region information, such as an organ of a subject and a lesion, which has been input by a user through his/her manual operation may be used. Further, an image processing apparatus of the present invention may further include a region extraction means that extracts an imaged region of the image from the image, and use the extracted imaged region information. For example, methods disclosed in Japanese Unexamined Patent Publication No. 2002-109548 and Japanese Unexamined Patent Publication No. 2003-6661, which are proposed by the applicant of the present application, may be used. In the methods, a thorax is automatically detected by performing template matching using a template that is substantially similar to the outline of an average cardiothorax, as reference.

The second processed signal extraction means in the image processing apparatus of the present invention may subtract the first processed signal from the image signal and also add the subject component to the image signal in an arbitrary order as long as the second processed signal, in which the first processed signal is subtracted from the image signal and the subject component is added to the image signal, is finally extractable. For example, the second processed signal extraction means may extract the second processed signal by subtracting the first processed signal from the image signal, and by adding the extracted subject component to the image signal after subtraction. Alternatively, the second processed signal extraction means may extract the second processed signal by subtracting the extracted subject component from the first processed signal to obtain a signal, and by subtracting the obtained signal from the image signal.

According to an image processing apparatus, an image processing method and an image processing program of the present invention, a first processed signal is extracted by extracting an input spatial frequency component corresponding to a periodic pattern by performing filtering processing on an image signal. Further, a subject component of an image, and the subject component being included in the first processed signal, is extracted from the extracted first processed signal. Further, a second processed signal is extracted by subtracting the first processed signal from the image signal and also by adding the extracted subject component to the image signal. Therefore, it is possible to extract a subject component from the frequency component corresponding to the periodic pattern, and to restore the subject component to the current image. Hence, it is possible to remove only the periodic pattern, such as moire, while a component representing the subj ect, which is important information for diagnosis, is maintained. Consequently, it is possible to obtain, as the second process image, a high quality image appropriate for diagnosis based on the image.

BRIEF DESCRIPTION OF THE DRAWINGS

Figure 1 is a schematic diagram illustrating a radiography apparatus;
Figure 2 is a diagram illustrating a radiographic image obtained by imaging with a grid;
Figure 3 is a perspective view illustrating an example of a radiographic image readout apparatus;
Figure 4 is a diagram illustrating a relationship between scan directions and an image to be read out;
Figure 5 is a schematic diagram illustrating the configuration of an image processing apparatus according to a first embodiment of the present invention;
Figure 6 is a diagram illustrating an example of a lookup table for extracting a high contrast component from a first processed image; and
Figure 7 is a schematic diagram illustrating the configuration of an image processing apparatus according to a modified example of the first embodiment.

DESCRIPTION OF THE PREFERRED EMBODIMENTS

Hereinafter, embodiments of the present invention will be described with reference to drawings. In the following embodiments, a case in which a periodic pattern suppression processing apparatus according to the present invention is used in a radiographic image readout apparatus will be described. Alternatively, the periodic pattern suppression processing apparatus may be used in an image processing apparatus or the like for suppressing a periodic pattern included in a photographic image that was obtained in ordinary photography using a digital camera or the like when photography was performed through a window screen, a blind or the like. Here, the radiographic image readout apparatus reads out, as a digital image signal, a radiographic image of a human body recorded on a storable phosphor sheet by scanning the radiographic image with a laser beam.

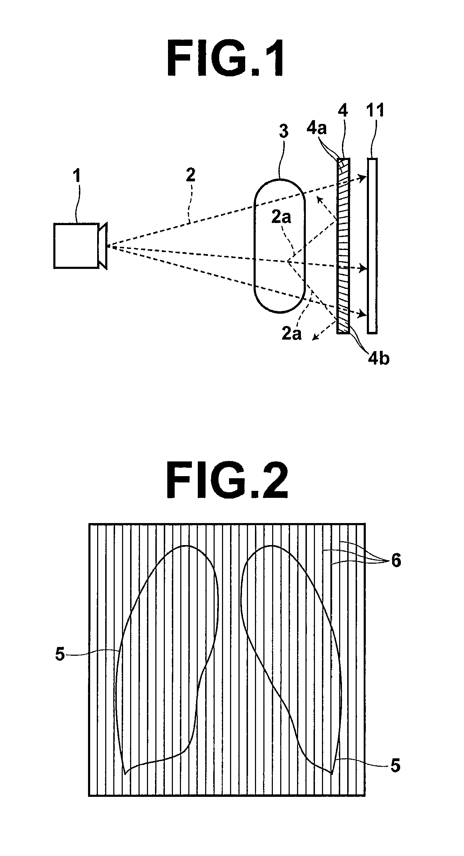

Figure 1 is a schematic diagram illustrating a radiography apparatus. Radiation 2 is output from a radiation source 1, and passes through a subject 3, and reaches a static grid (hereinafter, simply referred to as "grid") 4. In the grid 4, lead 4a, which absorbs the radiation 2, and aluminum 4b, which passes the radiation 2, are alternately arranged, for example, at a pitch of about 4 line/mm. Further, the lead 4a is set in such a manner that the inclination of the lead 4a is slightly different depending on its position so that the radiation 2 passes through the aluminum 4b, and enters a storable phosphor sheet 11.

Therefore, the radiation 2 that has passed through the subject 3 is absorbed by the lead 4a, and does not reach the storable phosphor sheet 11. However, the radiation 2 passes through the aluminum 4b, and reaches the storable phosphor sheet 11. A grid image of a stripe pattern of 4 line/mm is recorded on the storable phosphor sheet 11 together with a subject image. Meanwhile, scattered radiation 2a, which is scattered in the subject 3, is absorbed by the lead 4a, which is set in such a manner to be inclined depending on its position, or reflected by the surface of the grid 4. Therefore, the scattered radiation 2a does not reach the storable phosphor sheet 11. Hence, the storable phosphor sheet 11 can record a sharp radiographic image with a little amount of scattered radiation 2a irradiating the storable phosphor sheet 11. Figure 2 is a diagram illustrating an example of a radiographic image of a subject image 5 and a grid image 6. The radiographic image is stored and recorded on the storable phosphor sheet 11 when radiography is performed by using the radiography apparatus illustrated in Figure 1.

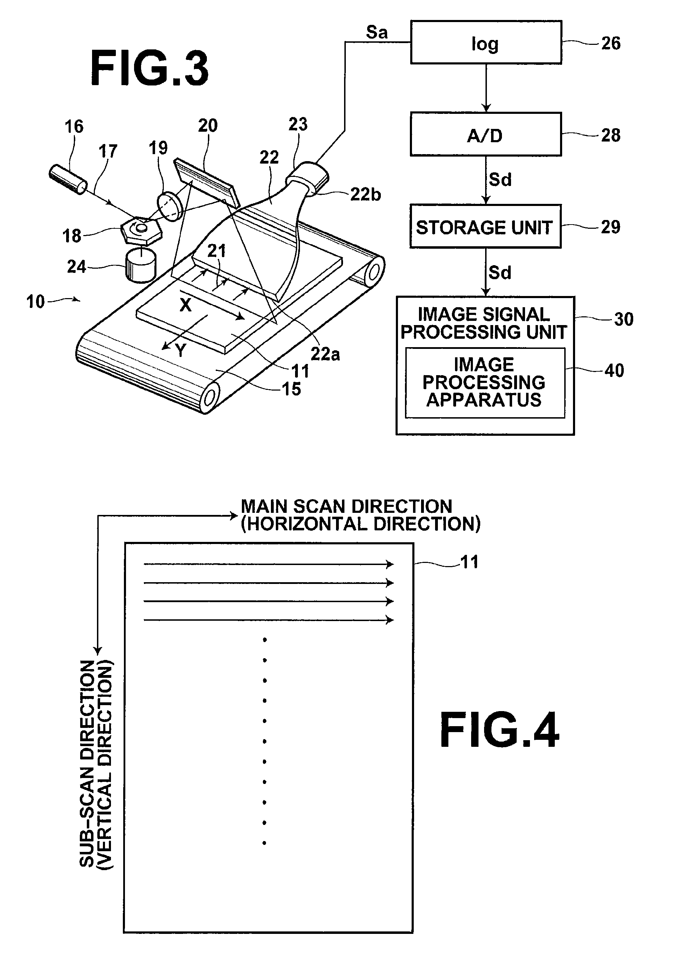

Figure 3 is a diagram illustrating a perspective view and a functional block diagram of a radiographic image readout apparatus in combination. The storable phosphor sheet 11 is set at a predetermined position of a readout unit 10, and conveyed by a sheet conveyance means 15, such as an endless belt, which is driven by a drive means (not illustrated). The storable phosphor sheet 11 is conveyed (sub-scanned) in the direction of arrow Y, for example, at a scan pitch of 10 line/mm. Meanwhile, a light beam 17 is output from a laser beam source 16, and reflected by a rotary polyhedral mirror 18 toward a condensing lens 19, such as an fθ lens. The rotary polyhedral mirror 18 is driven by a motor 24, and rotates at high speed in the direction of an arrow. After the light beam 17 passes through the condensing lens 19, the light beam 17 is reflected by a mirror 20 toward the storable phosphor sheet 11. The storable phosphor sheet 11 is main-scanned by the light beam 17 in the direction of arrow X, which is at a substantially right angle to the sub-scan direction (the direction of arrow Y).

When the storable phosphor sheet 11 is illuminated with the light beam 17, stimulated emission light 21 in an amount corresponding to radiographic image information stored and recorded at an illuminated position of the storable phosphor sheet 11 is emitted from the position. The stimulated emission light 21 enters a light guide 22 from an incident end surface 22a of the light guide 22, and repeats total reflection in the light guide 22, and is output from an output end surface 22b of the light guide 22. The output stimulated emission light 22 is received by a photomultiplyer 23, and converted into analog image signal Sa by photoelectric conversion.

After the analog image signal Sa is logarithmically amplified by a log amplifier 26, the analog image signal Sa is sampled with a sampling interval corresponding to a spatial frequency of fs = 10 cycle/mm and digitized by an A/D converter 28, and digital image signal Sd (hereinafter, simply referred to as "image signal Sd") is output. The image signal Sd represents radiographic image information obtained by two-dimensionally scanning the storable phosphor sheet 11. As illustrated in Figure 4, the radiographic image information is obtained by moving the storable phosphor sheet 11 in a sub-scan direction (vertical direction) while the storable phosphor sheet 11 is scanned with the light beam 17 in a main scan direction (horizontal direction). The image signal Sd obtained in this manner includes information about a grid image 6 corresponding to the grid 4 in addition to information about the radiographic image 5 corresponding to the subject 3.

After the image signal Sd is temporarily stored in a storage unit 29, the image signal Sd is input to an image signal processing unit 30. The image signal processing unit 30 includes an image processing apparatus 40 for performing an image processing method in the present invention.

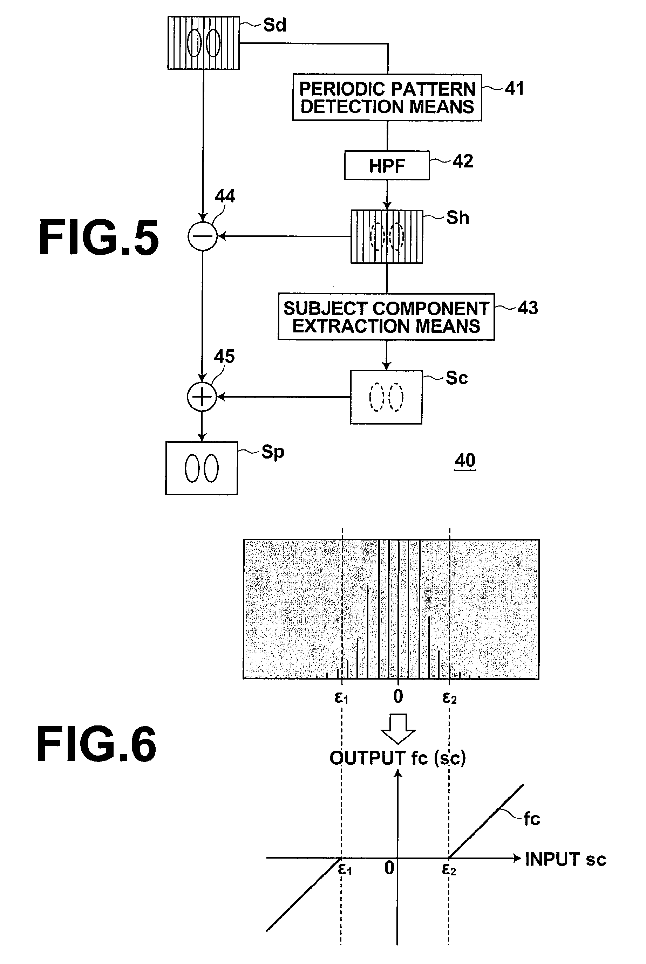

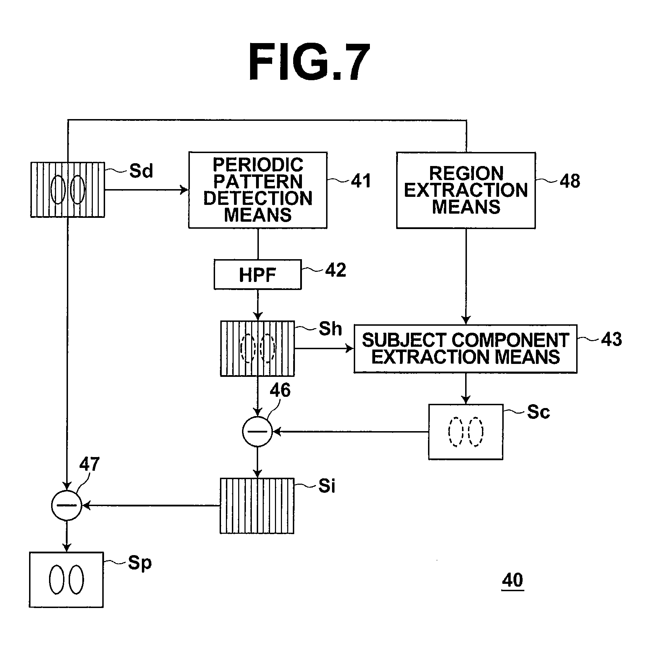

Figure 5 is a schematic block diagram illustrating the configuration of the image processing apparatus 40 in the first embodiment. Figure 6 is a diagram for explaining subject component extraction processing in the embodiment of the present invention. Figure 7 is a schematic block diagram illustrating the configuration of the image processing apparatus 40 in a modified example of the embodiment of the present invention. The image processing apparatus 40 according to the embodiment of the present invention will be described with reference to Figure 5 through Figure 7.

The image processing apparatus 40 in the embodiment of the present invention is an image processing apparatus that suppresses a spatial frequency component corresponding to a periodic pattern included in an image. The image processing apparatus 40 includes a periodic pattern detection means 41, a first processed signal extraction means 42, a subject component extraction means 43, and secondprocessed signal extractionmeans 44, 45. The periodic pattern detection means 41 detects, in an image signal, a spatial frequency component corresponding to a periodic pattern. The first processed signal extraction means 42 extracts a first processed signal by extracting the detected spatial frequency component corresponding to the periodic pattern by performing filtering processing on the image signal. The subject component extraction means 43 extracts, from the extracted first processed signal, a subject component of the image, and the subject component being included in the first processed signal. The second processed signal extraction means 44, 45 extract a second processed signal by subtracting the first processed signal from the image signal and also by adding the extracted subject component to the image signal.

An image processing program in an embodiment of the present invention and data to which the image processing program refers are stored in the storage unit 29 when the image processing program is installed, and loaded in a memory included in the storage unit 29 when the image processing program is started. The image processing program defines periodic pattern detection processing, first processed signal extraction processing, subject component extraction processing and second processed signal extraction, as processing performed by a central processing unit of the image processing unit 30, which constitutes the image processing apparatus 40. The central processing unit executes each of the aforementioned kinds of processing based on the program. Accordingly, the central processing unit of the image processing unit 30 functions as the periodic pattern detection means 41, the first processed signal extraction means 42, the subject component extraction means 43, and the second processed signal extraction means 44, 45.

As illustrated in Figure 5, the periodic pattern detection means 41 reads out image signal Sd from the storage unit 29, and detects a spatial frequency corresponding to a periodic pattern by using a known method, as disclosed in Patent Document 1 and Japanese Unexamined Patent Publication No. 2003-233818, which were filed by the applicant of the present application. The first processed signal extraction means 42 includes a high-pass filter or a band-pass filter, which removes a spatial frequency band. The first processed signal extraction means 42 performs filtering processing in such a manner to pass the detected spatial frequency band corresponding to the periodic pattern. Here, the first processed signal extraction means 42 extracts first processed signal Sh by performing, on image signal Sd from the storage unit 29, one-dimensional high-pass filter (HPF) processing in both of a horizontal direction and a vertical direction. The first processed signal extraction means 42 may use any filter as long as first processed signal Sh is extracted by performing filtering processing on image signal Sd in such a manner to pass the spatial frequency band corresponding to the periodic pattern. Filtering processing may be performed by a two-dimensional filter or filters, or by a two-dimensional filter.

The subject component extraction means 43 extracts, as the subject component, a high contrast component exceeding a predetermined threshold from first processed signal Sh. As will be described later, the subject component extraction means 43 obtains first processed signal Sh, and refers to lookup table fs[sc] based on contrast sc of the first processed signal Sh. The subject component extraction means 43 extracts, as the subject component, signal Sc representing a high contrast component from the first processed signal.

With reference to Figure 6, processing by the subject component extraction means 43 to extract, as a subject component, a high contrast component included in the first processed signal will be described.

The subject component extraction means 43 analyzes the contrast of first processed signal Sh, and performs, based on lookup table fs[sc], function processing corresponding to the contrast of the first processed signal Sh. The upper diagram of Figure 6 is a histogram representing the contrast of first processed signal Sh, and the lower diagram of Figure 6 is a graph of a function used by the subject component extraction means 43. In the graph illustrated in the lower diagram of Figure 6, the horizontal axis is input sc (specifically, contrast of first processed signal Sh), and the vertical axis is output (specifically, fs[sc]). In the graph of fs[sc], output = 0 when $\varepsilon_1 \leq sc \leq \varepsilon_2$, and the output increases or decreases in proportion to signal Sh when $sc > \varepsilon 2$ or $sc < \varepsilon 1$. In other words, only a component less than $\varepsilon 1$ and a component less than $\varepsilon 2$ in the upper diagram of Figure 6 are extracted as high contrast components. Further, in the embodiment of the present invention, the absolute value of output fs[sc]) is larger as contrast sc of first processed signal Sh is larger, as illustrated in lookup table fs in the lower diagram of Figure 6.

The lower diagram of Figure 6 corresponds to the following expression:
[Expression 1]

$$fs[sc] = \begin{cases} sc - \varepsilon_1 & (sc < \varepsilon_1) \\ 0 & (\varepsilon_1 \leq sc \leq \varepsilon_2) \\ sc - \varepsilon_2 & (sc > \varepsilon_2) \end{cases} \cdots (1)$$

In the embodiment of the present invention, lookup table fs[sc] illustrated in the lower diagram of Figure 6 outputs a difference between contrast sc and threshold $\varepsilon_1$ or a difference between contrast sc and $\varepsilon_2$. Such correspondence between an input signal and an output signal may be set in an arbitrary manner by using a known method. For example, an input signal maybe used directly as an output signal. Alternatively, appropriate weighting may be performed on contrast sc to get an output signal.

The second processed signal extraction means in the embodiment of the present invention includes a subtractor 44 and an adder 45. The subtractor 44 subtracts first processed signal Sh from image signal Sd. After then, the adder 45 adds signal Sc representing a high contrast component. Accordingly, the second processed signal extraction means extracts second processed signal Sp representing an image in which a periodic pattern has been suppressed. Here, Second Processed Signal Sp = (Image Signal Sd - First Processed Signal Sh + Signal Sc, which corresponds to a subject component in the first processed signal). Further, an image corresponding to the second processed signal Sp is displayed on a display means, such as a monitor (not illustrated), or output to an output means, such as a printer (not illustrated).

According to the embodiment of the present invention, it is possible to extract a subject component from the frequency component corresponding to the periodic pattern, and to restore the extracted subject component to the current image. Hence, it is possible to remove only the periodic pattern, such as moire, while a component that represents the subject, and that is included in the frequency band of the periodic pattern such as moire, is maintained. Consequently, it is possible to obtain, as the second process image, a high quality image appropriate for diagnosis based on the image.

Patent Documents 1 through 3 in the related art disclose removal of a grid image extracted as a high frequency component. When these methods are applied to moire that is detected as a low frequency component, many subject components included in the low frequency component are removed together with the moire component. Therefore, the subject component in the image after processing deteriorates. Hence, it has been difficult to maintain an image quality appropriate for diagnosis based on the image. However, when the image processing method according to the embodiment of the present invention is applied to moire, which is detected as a low frequency component, it is possible to appropriately remove only the moire component while many subject components included in the low frequency component are maintained, compared with the techniques in the related art, because it is possible to generate a second processed image in which a component that represents a subject, and that is included in a frequency band of a periodic pattern, such as moire, is added.

Further, the inventor of the present invention has found, after diligent research, that the contrast of moire generated as a folding distortion of a periodic pattern of a grid tends to be relatively low, compared with that of a subject obtained by actual imaging. Therefore, according to the embodiment of the present invention, it is possible to remove only a low contrast component from a frequency component that corresponds to the periodic pattern, such as a moire stripe, which is a folding distortion, in an concentrated manner by using a high contrast component as a subject component. Accordingly, it is possible to obtain a high quality image in which the subject component is appropriately maintained.

A modified example of the embodiment of the present invention will be described.

In the embodiment of the present invention, subtraction processing by the subtractor 44 and addition processing by the adder 45 may be performed in any order as long as signal Sp, which is represented by Second Processed Signal Sp = (Image Signal Sd - First Processed Signal Sh + Signal Sc, which corresponds to a subject component in the first processed signal), is obtainable. Any method may be used to restore the signal Sc, which corresponds to the subject component in the first processed signal, to obtain the second processed signal Sp. For example, as in the aforementioned embodiment, the second processed signal extraction means may extract the second processed signal by subtracting the first processed signal from the image signal, and by adding the extracted subject component to the image signal after subtraction. Alternatively, for example, the second processed signal extraction means may include a subtractor 46 and a subtractor 47, as illustrated in Figure 7. In this case, the second processed signal Sp may be extracted in the following manner. The subtractor 46 subtracts signal Sc representing the extracted subject component from first processed signal Sh to obtain signal Si, and the subtractor 47 subtracts the signal Si from the image signal Sd to extract the second processed signal Sp.

In lookup table fs in the aforementioned embodiment, the values of threshold $\varepsilon_1$ and/or $\varepsilon_2$ may be set at arbitrary values based on an imaged region, an imaging condition, or the like. For example, $\varepsilon_1$ and $\varepsilon_2$ may be set in such a manner that the upper (lower) N% (N = 3, 5, or the like) of the histogram in the upper diagram of Figure 6 is extracted. Alternatively, standard deviation $\sigma$ of the histogram may be calculated, and $\varepsilon_1$ and $\varepsilon_2$ may be set as $\varepsilon_1 = \varepsilon_2 = \pm N \times \sigma$ (N = 1.5, 2.0, or the like). For example, a correspondence table in which a threshold is linked with each imaged region and each imaging condition may be stored in the storage unit 29 in advance. Further the subject component extraction means may identify, based on the correspondence table, a threshold condition linked with the imaged region or the imaging condition of an image to be processed. Further, the subject component extraction means may extract a high contrast component by using the identified threshold condition.

In that case, it is possible to appropriately extract a subject component based on a threshold condition corresponding to the imaged region and the imaging condition. Therefore, it is possible to appropriately extract the second processed signal. Consequently, it is possible to generate a higher quality processed image.

In lookup table fs in the aforementioned embodiment, different lookup table fs may be used depending on the imaged region, the imaging condition or the like. For example, lookup table fs maybe selectable, based on the imaged region, the imaging condition, or the like, from plural functions (for example, function fs, function fs1, function fs2, and the like). In this case, the imaged region and the imaging condition of the image may be obtained by using an imaging menu, such as the imaging condition and information about the imaged region, which is specified at an imaging apparatus of the original image. Alternatively, imaged region information, such as an organ of the patient and a lesion, which has been input by a user through a manual operation may be used. Since it is possible to appropriately extract the subject component based on the lookup table corresponding to the imaged region and the imaging condition, it is possible to appropriately extract the second processed signal. Consequently, it is possible to generate a higher quality processed image.

Further, the image processing apparatus 40 may further include a region extraction means 48 that extracts an imaged region of an image, as illustrated in Figure 7, and use the extracted imaged region information. As a method for extracting imaged region information, any method may be adopted as long as a region, a lesion or the like can be extracted. For example, methods disclosed in Japanese Unexamined Patent Publication No. 2002-109548 and Japanese Unexamined Patent Publication No. 2003-6661, which are proposed by the applicant of the present application, may be used. In the methods, a thorax is automatically detected by performing template matching using a template that is substantially similar to the outline of an average cardiothorax, as reference.

In such a case, the subject component extraction means 43 does not need a user's input operation of an imaged region, and can extract an imaged region based on the result of automatic extraction. Therefore, it is possible to appropriately extract a subject component based on the extracted imaged region. Hence, a user can easily generate a high quality processed image based on the imaged region. Further, when the subject component extraction means 43 automatically extracts an imaged region or an imaging condition from an imaging menu, and extracts a subject component based on the result of automatic extraction, the same effect is achievable.

In the embodiment of the present invention, the subject component may be any component besides the high contrast component as long as the subject component represents the component of a subject.

In the embodiments of the present invention, a case in which the present invention is applied to a radiographic image readout apparatus (CR: Computed Radiography) has been described. In the radiographic image readout apparatus, excitation light, such as a laser beam, is output to a storable phosphor sheet, and stimulated emission light is generated. Photoelectric conversion is performed on the stimulated emission light to obtain an image signal. The present invention is not limited to the aforementioned embodiments, and may be applied to any type of radiographic image readout apparatus using a grid. For example, the present invention may be applied to a radiographic image readout apparatus using a radiation solid-state detector (hereinafter referred to as a radiation solid-state detector of "light conversion type and indirect conversion type"), and in which plural photoelectric conversion elements, each corresponding to a pixel, are two-dimensionally formed on an insulation substrate. A phosphor layer (scintillator) that converts radiation carrying image information into visible light by irradiation with the radiation is deposited and constitutes the radiation solid-state detector formed on the radiation image readout apparatus. Further, the present invention may be applied to a radiography apparatus using a radiation solid-state detector (hereinafter referred to as a radiation solid-state detector of "direct conversion type"). Plural charge collecting electrodes, each corresponding to a pixel, are two-dimensionally formed on an insulation substrate in a radiographic image readout apparatus. A radiation conductor that generates charges carrying image information by irradiation with radiation carrying the image information is deposited, and constitutes the radiation solid-state detector formed on the two-dimensional image readout apparatus.

Further, the present invention may be applied to a radiographic image readout apparatus using various kinds of radiation solid-state detector. As a radiation solid-state detector of light conversion type, radiation solid-state detectors disclosed, for example, in Japanese Unexamined Patent Publication No. 59(1984)-211263, Japanese Unexamined Patent publication No. 2(1990)-164067, PCT International Publication No. WO92/06501, Signal, noise, and read out considerations in the development of amorphous silicon photodiode arrays for radiotherapy and diagnostic x-ray imaging, L.E.Antonuket.al, University of Michigan, R.A.Street Xerox, PARC, SPIE Vol.1443 Medical Imaging V; Image Physics (1991), pp. 108-119, and the like may be adopted.

Meanwhile, as a radiation solid-state detector of direct conversion type, radiation solid-state detectors disclosed for example in (i) a radiation solid-state detector, the thickness of which in the transmission direction of radiation is set about ten times as thick as an ordinary one (MATERIAL PARAMETERS IN THICK HYDROGENATED AMORPHOUS SILICON RADIATION DETECTORS, Lawrence Berkeley Laboratory.University of California, Berkeley. CA 94720 XeroxParc.Palo Alto.CA 94304), or (ii) a radiation solid-state detector, in which two or more layers are deposited in the transmission direction of radiation with a metal plate therebetween (Metal/Amorphous Silicon Multilayer Radiation Detectors, IEE TRANSACTIONS ON NUCLEAR SCIENCE. VOL.36. NO.2. APRIL 1989), or (iii) a radiation solid-state detector using CdTe or the like (Japanese Unexamined Patent Publication No. 1(1989)-216290) or the like may be adopted.

Each of the aforementioned embodiments is only an example, and all of the descriptions should not be used to interpret the technical scope of the present invention in a limitedmanner. Further, the system configuration, the hardware configuration, the process flow, the module configuration, the specific content of processing and the like may be modified in various manners without departing from the gist of the present invention. Such modifications remain in the technical scope of the present invention.

## Claims

1. An image processing apparatus that suppresses a spatial frequency component corresponding to a periodic pattern included in an image, the apparatus comprising:

   a first processed signal extraction means that extracts a first processed signal by extracting an input spatial frequency component corresponding to the periodic pattern by performing filtering processing on the image signal;
   a subject component extraction means that extracts, from the extracted first processed signal, a subject component of the image, and the subject component being included in the first processed signal; and
   a second processed signal extraction means that extracts a second processed signal by subtracting the first processed signal from the image signal and also by adding the extracted subject component to the image signal.

2. The image processing apparatus, as defined in Claim 1, wherein the subject component extraction means extracts, from the first processed signal, a high contrast component exceeding a predetermined threshold, as the subject component.

3. The image processing apparatus, as defined in Claim 1 or 2, wherein the threshold differs depending on an imaged region or an imaging condition of the image.

4. The image processing apparatus, as defined in any one of Claims 1 to 3, the apparatus further comprising:

   a region extraction means that extracts an imaged region of the image from the image.

5. The image processing apparatus, as defined in any one of Claims 1 to 4, the apparatus further comprising:

   a periodic pattern detection means that detects, in the image signal, the spatial frequency component corresponding to the periodic pattern, and that inputs the detected spatial frequency component into the first processed signal extraction means.

6. The image processing apparatus, as defined in any one of Claims 1 to 5, wherein the second processed signal extraction means extracts the second processed signal by subtracting the first processed signal from the image signal, and by adding the extracted subject component to the image signal after subtraction.

7. The image processing apparatus, as defined in any one of Claims 1 to 5, wherein the second processed signal extraction means extracts the second processed signal by subtracting the extracted subject component from the first processed signal to obtain a signal, and by subtracting the obtained signal from the image signal.

8. An image processing method that suppresses a spatial frequency component corresponding to a periodic pattern included in an image, the method comprising the steps of:

   extracting a first processed signal by extracting an input spatial frequency component corresponding to the periodic pattern by performing filtering processing on the image signal;
   extracting, from the extracted first processed signal, a subject component of the image, and the subject component being included in the first processed signal; and
extracting a second processed signal by subtracting the first processed signal from the image signal and also by adding the extracted subject component to the image signal.

9. An image processing program that suppresses a spatial frequency component corresponding to a periodic pattern included in an image, the program causing a computer to function as:

a first processed signal extraction means that extracts a first processed signal by extracting an input spatial frequency component corresponding to the periodic pattern by performing filtering processing on the image signal;
a subject component extraction means that extracts, from the extracted first processed signal, a subject component of the image, and the subject component being included in the first processed signal; and
a second processed signal extraction means that extracts a second processed signal by subtracting the first processed signal from the image signal and also by adding the extracted subject component to the image signal.

# FIG.1

# FIG.2

# FIG.3

Sa → log — 26

A/D — 28

↓ Sd

STORAGE UNIT — 29

↓ Sd

IMAGE SIGNAL PROCESSING UNIT — 30

IMAGE PROCESSING APPARATUS — 40

MAIN SCAN DIRECTION (HORIZONTAL DIRECTION)

SUB-SCAN DIRECTION (VERTICAL DIRECTION)

11

# FIG.4

EP 2 689 725 A1

**FIG.5**

Sd → PERIODIC PATTERN DETECTION MEANS ~41

PERIODIC PATTERN DETECTION MEANS → HPF ~42

HPF → Sh

Sh → (−) 44

44 (−) ← Sh

Sh → SUBJECT COMPONENT EXTRACTION MEANS ~43

SUBJECT COMPONENT EXTRACTION MEANS → Sc

Sc → (+) 45

45 (+) → Sp

40

**FIG.6**

ε₁    0    ε₂

⇩

OUTPUT fc (sc)

fc

ε₁    0    ε₂    INPUT sc

13

# FIG.7

| INTERNATIONAL SEARCH REPORT | International application No. |
| --- | --- |
| | PCT/JP2012/002010 |

A. CLASSIFICATION OF SUBJECT MATTER
*A61B6/00*(2006.01)i, *G06T5/00*(2006.01)i, *G06T5/20*(2006.01)i

According to International Patent Classification (IPC) or to both national classification and IPC

B. FIELDS SEARCHED

Minimum documentation searched (classification system followed by classification symbols)
A61B6/00, G06T5/00, G06T5/20

Documentation searched other than minimum documentation to the extent that such documents are included in the fields searched
Jitsuyo Shinan Koho           1922-1996   Jitsuyo Shinan Toroku Koho   1996-2012
Kokai Jitsuyo Shinan Koho     1971-2012   Toroku Jitsuyo Shinan Koho   1994-2012

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2010-239480 A  (Fujifilm Corp.),<br>21 October 2010 (21.10.2010),<br>entire text; all drawings<br>& US 2010/0246922 A1 | 1-9 |
| A | JP 2010-104517 A  (Canon Inc.),<br>13 May 2010 (13.05.2010),<br>entire text; all drawings<br>& US 2010/0104165 A1 | 1-9 |
| A | JP 2009-195512 A  (Fujifilm Corp.),<br>03 September 2009 (03.09.2009),<br>entire text; all drawings<br>& US 2009/0214130 A1 | 1-9 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 23 May, 2012 (23.05.12) | 05 June, 2012 (05.06.12) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**EP 2 689 725 A1**

**INTERNATIONAL SEARCH REPORT**

| International application No. |
| --- |
| PCT/JP2012/002010 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2003-174590 A  (Canon Inc.),<br>20 June 2003 (20.06.2003),<br>entire text; all drawings<br>(Family: none) | 1-9 |
| A | JP 2002-330343 A  (Canon Inc.),<br>15 November 2002 (15.11.2002),<br>entire text; all drawings<br>(Family: none) | 1-9 |

Form PCT/ISA/210 (continuation of second sheet) (July 2009)

**16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003150954 A
- JP 3445258 B
- JP 2010240259 A
- JP 2002109548 A
- JP 2003006661 A

- JP 2003233818 A
- JP 59211263 A
- JP 2164067 A
- WO 9206501 A
- JP 1216290 A

**Non-patent literature cited in the description**

- **L.E.ANTONUK.** University of Michigan, R.A.Street Xerox, PARC, SPIE Vol.1443 Medical Imaging V. *Image Physics,* 1991, vol. 143, 108-119

- Metal/Amorphous Silicon Multilayer Radiation Detectors. *IEE TRANSACTIONS ON NUCLEAR SCIENCE,* April 1996, vol. 36 (2.